# EUROPEAN PATENT APPLICATION

(11) **EP 3 127 519 A1**
(43) Date of publication of application: **08.02.2017**
(21) Application number: 15772907.0
(22) Date of filing: 12.03.2015
(51) Int. Cl.: A61F 13/49, A61F 13/15, A61F 13/511, A61F 13/539

(54) **ABSORBENT ARTICLE**

(30) Priority: 04.04.2014 JP 2014078050
(71) Applicant: Unicharm Corporation, Ehime 799-0111 (JP)
(72) Inventor: KIMURA, Akihiro, Kanonji-shi Kagawa 769-1602 (JP); DETANI, Ko, Kanonji-shi Kagawa 769-1602 (JP); KAMEDA, Noritomo, Kanonji-shi Kagawa 769-1602 (JP)
(74) Representative: Peter, Julian
(86) International application number: PCT/JP2015/057346
(87) International publication number: WO 2015/151757

(57) **Abstract**

The present invention provides an absorbent article that has flexible cushioning properties, favorable rebound when compressed, superior collection of waste, and superior liquid migration properties from a surface sheet to an absorbent element. The absorbent article has: a surface sheet (2), which has a textured structure used at the skin surface side of the absorbent article, and has first protrusions (21) protruding to the skin surface side and second protrusions (22) protruding to the clothing side at the reverse of the skin surface side; and an absorbent element (4) disposed at a position overlapping the surface sheet (2) in a plan view. The surface sheet (2) and the absorbent element (4) are joined in a planar manner at a join section at the apices (22T) of the second protrusions (22).

## Description

### Technical Field

The present invention relates to an absorbent article in which a front sheet with a protrusion-recess structure and an absorbing element are joined.

### Background Art

Absorbent articles such as disposable diapers, sanitary napkins or panty liners are usually used in direct contact with the skin of the user, and some are even worn for long periods. An absorbent article must therefore be gentle on the skin, and have excellent absorption properties and uptake retentivity for fluids, such as urine or menstrual blood, excreta and the like.

In order to obtain a front sheet to constitute the skin side surface of an absorbent article, the front sheet having a soft cushioning property, satisfactory restoration when pressed, excellent scavenging properties for excreta, and especially excellent uptake of fluids, PTL 1 discloses a technique using a protrusion-recess nonwoven fabric provided with numerous alternating first protrusions that protrude to a first side and second protrusions that protrude to a second side opposite the first side, as a nonwoven fabric to compose the front sheet.

In order to promote smooth migration of fluids such as excreta, the protrusion-recess nonwoven fabric disclosed in PTL 1 is constructed with lower fiber density on the second side than on the first side at the top sections of the first protrusions, and with essentially equal fiber density on the first side and second side at the top sections of the second protrusions, the fiber density of the protrusion-recess nonwoven fabric in the thickness direction varying from sparse to dense from the skin side toward the absorbent body side.

### Citation List

### Patent Literature

[PTL 1] Japanese Unexamined Patent Publication No. 2012-144835

### Summary of Invention

### Technical Problem

However, when such a protrusion-recess nonwoven fabric is joined with a nonwoven fabric or tissue on the skin side of an absorbing element, the fiber density is highest on the protrusion-recess nonwoven fabric side (i.e., the fiber density on the second side of the top sections of the second protrusions) at both joining sections, and it is therefore resistant to deformation and the cushioning property or restoration after pressing are poor. In addition, since the form of the joining sections is as small point joints with small joining area, this has created a problem of poor ease of uptake of body fluids such as urine and menstrual blood, or in other words, poor fluid migration properties.

It is therefore an object of the present invention to provide an absorbent article with a soft cushioning property, satisfactory restoration when pressed, and excellent scavenging properties for excreta, as well as excellent fluid migration properties from the front sheet to the absorbing element.

### Solution to Problem

The absorbent article of the present invention is an absorbent article having a front sheet with a protrusion-recess structure, to be used on the skin side of the absorbent article, the front sheet having first protrusions that protrude to the skin side and second protrusions that protrude to the clothing side on the opposite side from the skin side, and an absorbing element situated at a location overlapping with the front sheet in a plane view, wherein the front sheet and the absorbing element are joined with planar forms at the joining sections of the top sections of the second protrusions.

According to the absorbent article of the present invention, the joining sections between the front sheet with a protrusion-recess structure and the absorbing element are not point joints with small joining areas as in the prior art, but instead have planar forms, and therefore the joining area between the front sheet and absorbing element is larger compared to an absorbent article of the prior art, and the region in which body fluids such as urine and menstrual blood discharged onto the front sheet can migrate into the absorbing element is increased. Furthermore, when the joining sections have planar forms, in addition to allowing improved fluid migration properties as described above, it is possible to create an outer appearance with a protrusion-recess feel (three-dimensional feel) desired for a front sheet with a protrusion-recess structure, by the first protrusions that protrude to the skin side, and therefore an absorbent article provided with both an excellent function and satisfactory outer appearance as an absorbent article can be obtained.

### Advantageous Effects of Invention

According to the present invention it is possible to provide an absorbent article with a soft cushioning property, satisfactory restoration when pressed, and excellent scavenging properties for excreta, as well as excellent fluid migration properties from the front sheet to the absorbing element.

### Brief Description of Drawings

Fig. 1 is a perspective view of a disposable diaper as an embodiment of the absorbent article of the present invention.
Fig. 2 is a plan view of the disposable diaper of Fig. 1 in the deployed state.
Fig. 3 is a partial plan view of a front sheet to be used in an absorbent article of the present invention.
Fig. 4 is a partial cross-sectional view along cross-section Z-Z' of Fig. 3.
Fig. 5 is a schematic diagram of production equipment for a protrusion-recess nonwoven fabric to compose a front sheet.
Fig. 6 is an electron micrograph of the cut surface of the top section of a first protrusion in a protrusion-recess nonwoven fabric composing a front sheet to be used in the absorbent article of the present invention.
Fig. 7 is a schematic view for illustration of the distributed state of fiber density before and after joining between a protrusion-recess nonwoven fabric to compose a front sheet and an absorbing element.
Fig. 8 is an electron micrograph of the cut surface of a joining section between a protrusion-recess nonwoven fabric to compose a front sheet and a nonwoven fabric on the absorbing element side.
Fig. 9 is an electron micrograph of the cut surface of a joining section in the layered body sample of Example 1.
Fig. 10 is an electron micrograph of the cut surface of a joining section in the layered body sample of Example 2.

### Description of Embodiments

Preferred embodiments of the absorbent article of the present invention will now be described in detail with reference to the accompanying drawings.

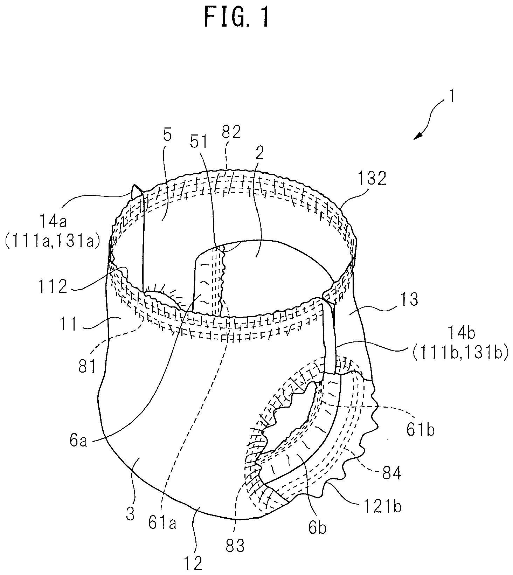

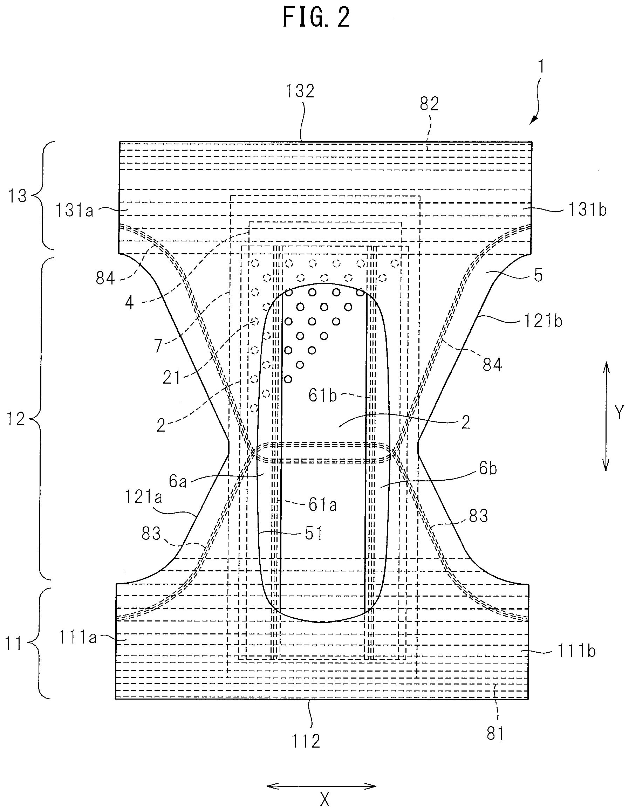

Fig. 1 is a perspective view of a disposable diaper as an embodiment of the absorbent article of the present invention, and Fig. 2 is a plan view of the disposable diaper of Fig. 1 in the deployed state. As shown in Fig. 1 and Fig. 2, the disposable diaper 1 as an embodiment of the present invention has a front section 11 which is contacted with the abdominal region of the wearer, a middle section 12 which is contacted with the crotch region of the wearer, and a back section 13 which is contacted with the gluteal region and/or back region of the wearer. As shown in Fig. 1, both edges 111 a, 111 b of the front section 11 and both edges 131 a, 131 b of the back section 13 are joined together at the joining sections 14a, 14b, whereby a waist opening is formed by the edge 112 of the front section 11 and the edge 132 of the back section 13 and leg openings are formed by both edges 121a, 121b of the middle section 12, the disposable diaper 1 having a pants-type shape.

As shown in Fig. 1 and Fig. 2, the disposable diaper 1 comprises a liquid-permeable front sheet 2, a liquid-impermeable back sheet 3, an absorbing element 4 provided between the front sheet 2 and the back sheet 3, a liquid-impermeable cover sheet 5, liquid-impermeable leak-resistant cuffs 6a, 6b, a liquid-impermeable leak-resistant sheet 7, and elastic members 81, 82, 83, 84. The cover sheet 5 provided on the skin side surface of the front sheet 2 has an opening 51 formed at approximately the center, a portion of the front sheet 2 (the portion of the region where the absorbing element 4 is situated) being exposed from the opening 51 of the cover sheet 5 and, together with the cover sheet 5, constituting the skin side surface of the disposable diaper 1. Also, one edge of each of the leak-resistant cuffs 6a, 6b provided on both sides of the opening 51 of the cover sheet 5 is the anchored edge that is sandwiched and anchored between the front sheet 2 and cover sheet 5, while the other edge is the free edge that is exposed through the opening 51 of the cover sheet 5. At the free edges of the leak-resistant cuffs 6a, 6b there are provided elastic sections 61 a, 61 b that extend in the lengthwise direction Y of the disposable diaper 1, the leak-resistant cuffs 6a, 6b standing up toward the skin side of the wearer.

Also, as shown in Fig. 1 and Fig. 2, elastic members 81, 82, 83, 84 are provided between the back sheet 3 and the cover sheet 5, which have hourglass shapes of approximately equal dimensions. Elastic contractive force of the elastic members 81, 82 causes formation of waist gathers at the waist opening, and elastic contractive force of the elastic members 83, 84 causes formation of leg gathers (leg side cuffs) at the leg openings. The leg gathers can prevent leakage of excreta from the leg openings. Throughout the present description, the widthwise direction X is the widthwise direction of the disposable diaper 1 (absorbent article) in the deployed state in a plane view (the short direction), and the lengthwise direction Y is the lengthwise direction of the disposable diaper 1 (absorbent article) in the deployed state in a plane view (the front-back direction of the wearer), the widthwise direction X and lengthwise direction Y being mutually orthogonal in a plane view.

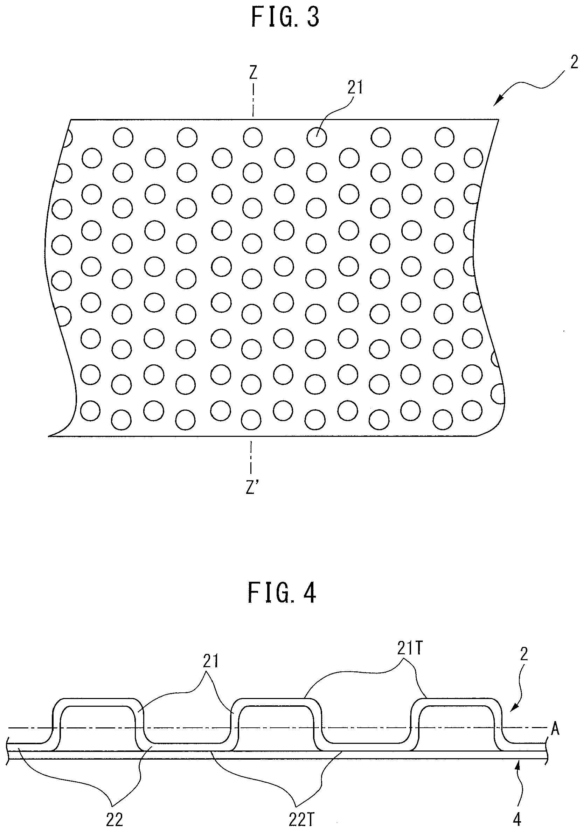

The front sheet and absorbing element to be used in the absorbent article of the present invention, and the form of their joining, will now be explained in detail. Fig. 3 is a partial plan view of a front sheet to be used in an absorbent article of the present invention. Also, Fig. 4 is a partial cross-sectional view along cross-section Z-Z' of Fig. 3, and in particular it shows the form of joining of the joining sections between the front sheet 2 made of a protrusion-recess nonwoven fabric, and the absorbing element 4.

For this embodiment, the front sheet 2 is a nonwoven fabric having a protrusion-recess structure in which there are formed first protrusions 21 that protrude to the skin side with respect to the center plane A that is parallel to the planar direction of the front sheet 2, and second protrusions 22 that protrude to the clothing side which is opposite from the skin side with respect to the center plane A. Also, the front sheet 2 and the absorbing element 4 are joined in a planar form at the joining sections of the top sections 22T of the second protrusions 22.

For this embodiment, the first protrusions 21 of the front sheet 2 have approximately cylindrical shapes in their appearance; however, the absorbent article of the present invention is not limited to these shapes. The first protrusions and second protrusions of the front sheet may be formed with any shapes such as elliptic cylindrical, columnar, such as polygonal columnar, conical such as circular conical or pyramidal, or truncated conical such as truncated circular conical or truncated pyramidal, and they may even be formed as hemispherical shapes in order to obtain a softer cushioning property. Of these, the particularly preferred shapes are shapes whose surfaces are formed flat on the skin side and clothing side, such as cylindrical, elliptic cylindrical, square columnar, truncated conical or truncated pyramidal. By forming the protrusions with such shapes, the protrusions can contact the skin surface of the wearer, allowing body fluids such as urine or menstrual blood that have adhered to the skin surface, to be taken up easier to the clothing side.

Moreover, the heights of the first protrusions and second protrusions (that is, the distances between each top section 21T, 22T and the center plane A in Fig. 4) are not particularly restricted, but from the viewpoint of cushioning property and feel, and prevent rewetting and the like, it may be in the range of, for example, 0.1 mm to 6.0 mm, and is preferably 0.2 mm to 5.0 mm and more preferably 0.2 mm to 4.0 mm.

The thickness of the front sheet is not particularly restricted and any desired thickness may be employed, but from the viewpoint of a suitable cushioning property and feel, preventing rewetting, and fluid migration properties and the like, it may be in the range of, for example, 0.1 mm to 10.0 mm, and is preferably 0.5 mm to 7.0 mm and more preferably 1.0 mm to 5.0 mm.

Also, the front sheet is preferably a nonwoven fabric. The fibers to be used in the nonwoven fabric are not particularly restricted and examples include natural fibers such as wool and cotton; and regenerated fibers such as rayon and acetate; as well as synthetic fibers composed using thermoplastic resins alone including polyolefins such as polyethylene (PE), polypropylene (PP) and polybutylene; ethylene-vinyl acetate copolymer (EVA); ethylene-ethyl acrylate copolymer; ethylene-acrylic acid copolymer; ionomers; polyesters such as polyethylene terephthalate (PET), polybutylene terephthalate (PBT), polytrimethylene terephthalate and polylactic acid; and polyamides such as nylon, or composite fibers such as core-sheath, side-by-side or sea-island fibers composed using multiple different types of the aforementioned thermoplastic resins. Core-sheath composite fibers preferably have a core-sheath structure wherein the core is polyethylene terephthalate (PET) and the sheath is polyethylene such as high-density polyethylene (HDPE) or low melting point polypropylene, and representative examples of such composite fibers include composite fibers with core/sheath = PET/HDPE, PET/PE, PP/PE and PP/low melting point PP. In addition, the form of such fibers is not particularly restricted, and there may be used hollow fibers; irregular cross-sectional fibers that are flat, Y-shaped, C-shaped or the like, latent crimping or developed crimping type solid crimped fibers, or splittable fibers that split under a physical load such as a water stream, heat or embossing. These fibers may be hydrophilic fibers or hydrophobic fibers; however, using hydrophobic fibers requires separate hydrophilicizing treatment with a hydrophilic treatment agent or the like. In addition, the fibers mentioned above may each be used alone, or two or more may be used in desired combinations.

The basis weight of the nonwoven fabric composing the front sheet is not particularly restricted but is preferably 10 g/m² to 100 g/m² and more preferably 15 g/m² to 50 g/m². If the basis weight is less than 10 g/m², sufficient surface strength may not be obtained, and tearing of the absorbent article may occur during use, while if it is greater than 100 g/m², excessive stiffness will result, potentially producing a feeling of unpleasantness or discomfort for the wearer during use. In addition, when the absorbent article is used for long periods, and the basis weight exceeds 50 g/m², body fluids such as urine and menstrual blood will be retained in the front sheet, continuously maintaining a sticky feel, and creating discomfort for the wearer.

The nonwoven fabric composing the front sheet may be a nonwoven fabric produced by any desired production method that is well known in the field, such as an air-through nonwoven fabric, spunlace nonwoven fabric, spunbond nonwoven fabric, thermal bond nonwoven fabric, meltblown nonwoven fabric or needle punching nonwoven fabric. However, for modification in order to more easily form planar joints with the absorbing element while varying the fiber density distribution in the protrusion-recess nonwoven fabric from sparse to dense in the thickness direction and obtain a structure that more easily takes up body fluids such as urine and menstrual blood, as explained below, it is particularly preferred to use a protrusion-recess nonwoven fabric produced by the production method described below.

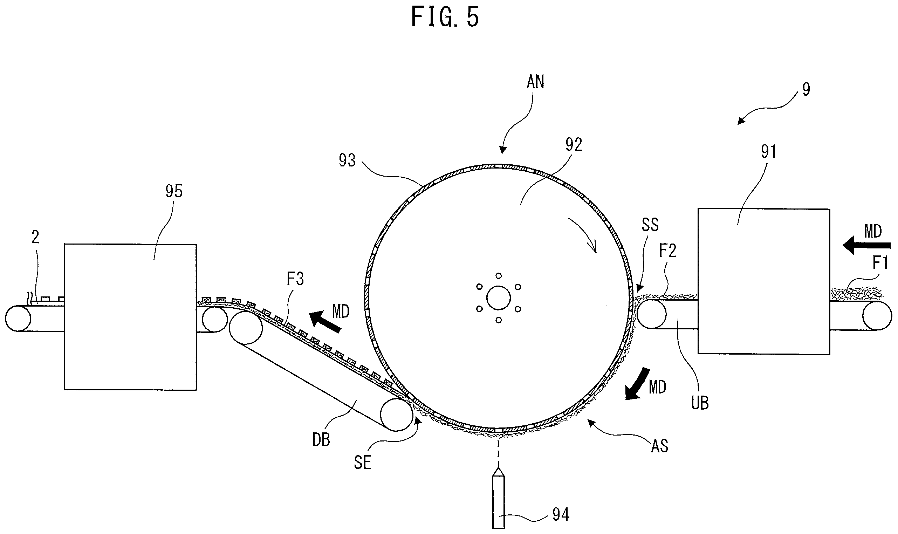

A method for producing a protrusion-recess nonwoven fabric to be used as a front sheet for an absorbent article of the present invention will now be explained with reference to Fig. 5.

Fig. 5 is a schematic diagram showing an example of production equipment 9 for production of a protrusion-recess nonwoven fabric to be used as a front sheet in an absorbent article of the present invention. The production equipment 9 comprises a carding machine 91 that opens the fibers F1 while adjusting the basis weight, a suction drum 92 and air jet nozzle 94 that form a protrusion-recess form in the fiber matrix F2 that has left the carding machine 91, and a heat setting machine 95 that heat sets the protrusion-recess fiber matrix F3 having the protrusion-recess form produced by the suction drum 92 and the air jet nozzle 94, to fix the protrusion-recess form. In Fig. 5, the fibers F1, fiber matrix F2, protrusion-recess fiber matrix F3 and the front sheet 2 comprising protrusion-recess nonwoven fabric, described below, are conveyed in the MD direction indicated by the arrow in Fig. 5, the MD direction coinciding with the lengthwise direction of the protrusion-recess nonwoven fabric.

In the method for producing a protrusion-recess nonwoven fabric using this type of production equipment 9, first the opened fibers F1 are supplied to the carding machine 91. At the carding machine 91, the supplied fibers F1 are further opened, and the basis weight of the fibers F1 is adjusted to the desired value.

The fiber matrix F2 that has left the carding machine 91 is then conveyed toward the surface of the suction drum 92. The suction drum preferably has its interior formed in a hollow or porous manner, and the interior may be brought to a negative pressure by any desired suction means, such as a blower or vacuum pump. One or a plurality of suction holes are formed on the outer peripheral surface of the suction drum, and external air can be suctioned near the outer peripheral surface through the suction holes. Also, there are no particular restrictions on the sizes of the suction holes, but they are preferably sizes that prevent the fiber matrix F2 from being sucked inside the suction drum, while ensuring the desired suction force.

The outer peripheral surface of the suction drum 92 is covered by a pattern plate 93 on at least a portion or on its entire periphery. The fiber matrix F2 that has left the carding machine 91 is supplied onto the pattern plate 93 that rotates together with the suction drum 92. The pattern plate 93 is, for example, punching metal provided with a prescribed pattern of a plurality of through-holes having shapes that are complementary with the first protrusions 21 of the front sheet 2.

In the suction drum 92, the fiber matrix F2 supplied onto the pattern plate 93 is suctioned by negative pressure from the suction holes formed in the outer peripheral surface of the suction drum 92, and is attracted onto the pattern plate 93 and in the through-holes. For this embodiment, the distance in the vertical direction (that is, the thickness direction) between the top sections 21T of the first protrusions 21 and the top sections 22T of the second protrusions 22 approximately matches the thickness of the pattern plate 93.

The suction drum 92 is designed so that it sucks at least in a region AS between a point SS on the outer peripheral surface on which the fiber matrix F2 is supplied from an upstream belt conveyor UB, and a point SE on the outer peripheral surface where the protrusion-recess fiber matrix F3 is supplied onto a downstream belt conveyor DB. The region AN outside of the suction region AS is preferably designed for no suction, from the viewpoint of the suction efficiency of the suction drum 92 and preventing inclusion of contaminants.

The fiber matrix F2 that has been adsorbed onto the pattern plate 93 on the outer peripheral surface of the suction drum 92 is blasted with a gas such as warm air by gas spray means such as an air jet nozzle 94. The air jet nozzle 94 is composed of one spray hole, or a plurality aligned in the widthwise direction of the fiber matrix F2, allowing warm air at a prescribed temperature to be sprayed at a prescribed wind speed. By appropriately adjusting the intervals for each spray hole, the distance between the spray holes and the fiber matrix F2, the shapes and sizes of the spray holes, etc., it is possible to blast the warm air essentially uniformly over the entire width of the fiber matrix F2, or locally onto a portion in the widthwise direction of the fiber matrix F2. The suction action of the suction drum 92 and the blasting action of the air jet nozzle 94 can form in the fiber matrix F2 shapes corresponding to the protrusion-recess form of the front sheet 2.

The temperature of the warm air blasted from the air jet nozzle is not particularly restricted, but from the viewpoint of formability, it is preferably set to be a higher temperature than the melting point of the material composing the fiber matrix F2, and for example, to a temperature 20°C to 70°C higher than the melting point. If the temperature of the warm air is too high the formed nonwoven fabric will be harder than necessary, and the temperature is preferably adjusted so as not to be too high. The wind speed of the warm air is not particularly restricted so long as it is a wind speed that allows the fiber matrix F2 to be formed into the prescribed protrusion-recess form, and for example, when the fiber matrix F2 has the basis weight and thickness of the nonwoven fabrics for absorbent articles of the examples of the present invention described below, the wind speed is in the range of 10.0 m/sec to 150.0 m/sec and preferably 15.0 m/sec to 100.0 m/sec. Since the temperature and wind speed of the warm air depends on the material and basis weight of the fibers used, the protrusion-recess form that is shaped, the placement of the air jet nozzles, the transport speed, etc., the optimal temperature and wind speed is preferably established by experimentation. As mentioned above, by blasting the fiber matrix F2 with warm air at a temperature higher than the melting point of the material composing the fiber matrix F2 at a prescribed wind speed, it is possible to precisely form the prescribed protrusion-recess form on the fiber matrix F2.

For this embodiment, the surface of the fiber matrix F2 on the side contacting the pattern plate 93 on the outer peripheral surface of the suction drum 92 is the surface on the skin side of the absorbent article; however, the present invention is not limited to this embodiment, and instead the surface on the side that is blasted with warm air may be the surface on the skin side of the absorbent article.

Furthermore, the sections of the fiber matrix F2 corresponding to the top sections 21T of the first protrusions 21 of the front sheet 2 have their fibers composing the fiber matrix F2 attracted to the suction drum 92 side by the suction action of the suction drum 92 and the blasting action of the air jet nozzle 94, whereby the fibers become maldistributed in the thickness direction of the fiber matrix F2. Due to such maldistribution of the fibers the protrusion-recess nonwoven fabric, the fiber density on the suction drum 92 side, i.e. the skin side of the absorbent article at the top sections 21 T of the first protrusions 21 is higher than the fiber density on the air jet nozzle 94 side, i.e. the clothing side of the absorbent article. Moreover, the sections corresponding to the top sections 22T of the second protrusions 22 of the fiber matrix F2 also have their fibers composing the fiber matrix F2 attracted to the suction drum 92 side by the suction action of the suction drum 92 and the blasting action of the air jet nozzle 94, those fibers becoming maldistributed in the thickness direction of the fiber matrix F2, and therefore at the top sections 22T of the second protrusions 22, the protrusion-recess nonwoven fabric has higher fiber density on the suction drum 92 side, i.e. the skin side of the absorbent article, than the fiber density on the air jet nozzle 94 side, i.e. the clothing side of the absorbent article.

As used throughout the present description, "fiber density" means the number of cut cross-sections of fibers per fixed area when a cut surface of the nonwoven fabric is observed in an enlarged view, and specifically, it is value obtained when a cut surface of the nonwoven fabric is observed in an enlarged view using a scanning electron microscope (for example, a VE-7800 Real Surface View microscope by Keyence Corp.), with the center in the thickness direction of the nonwoven fabric as the center of observation (that is, observation at a magnification allowing about 20 to 70 fiber cross-sections to be observed, which will normally be a magnification of about 20x to 100x), the number of cut cross-sections of fibers per fixed area (about 2 mm²) is counted, and the number of cut cross-sections is converted to the number of cut cross-sections of fibers per 1 mm² area. In the examples described below, the fiber density was measured at 3 locations and the mean value was recorded as the fiber density of the sample.

Throughout the present description, the phrase "fiber density on the skin side" means the fiber density at the section of the skin side where the thickness of the nonwoven fabric is halved, and "fiber density on the clothing side" means the fiber density of the remaining section as the clothing side.

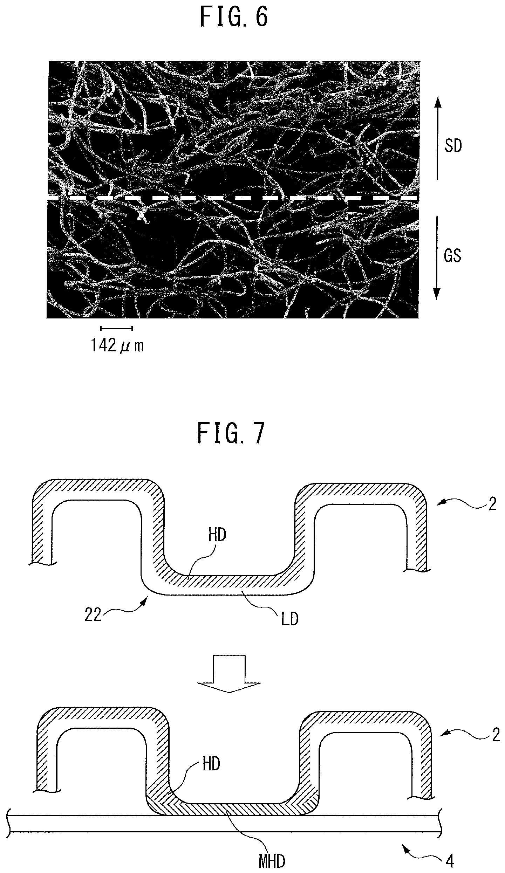

Fig. 6 is an electron micrograph of a cut surface of a top section 21T of a first protrusion 21 of the protrusion-recess nonwoven fabric. This photograph shows that the fiber density on the skin side SD is higher than the fiber density on the clothing side GD at the top section of the protrusion, as explained above. When the fiber density on the skin side is higher than the fiber density on the clothing side at the top sections 21T of the first protrusions 21, body fluids such as urine and menstrual blood supplied to the skin side of the protrusion-recess nonwoven fabric is less likely to pool in the top sections 21T of the first protrusions 21 and tends to more easily flow to the top section 22T side of the second protrusions 22, thereby allowing the body fluids to effectively penetrate at the joining sections of the large joining areas of the top sections 22T of the second protrusions 22, to thereby significantly reduce re-adhesion of body fluids onto the skin of the wearer.

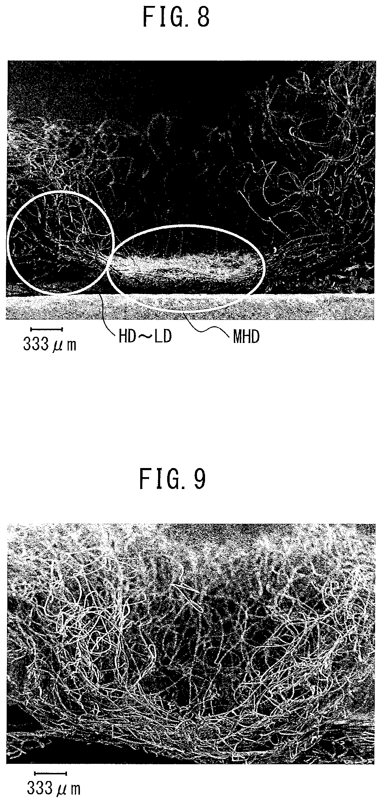

Fig. 7 is a schematic view of the state of fiber density before and after joining of a protrusion-recess nonwoven fabric, and Fig. 8 is an electron micrograph of the cut surface of a top section 22T of a second protrusion 22 after joining. As shown in Fig. 7, if the fiber density on the skin side is higher than the fiber density on the clothing side at the top sections 22T of the second protrusions 22 of the protrusion-recess nonwoven fabric (see high fiber density section HD on skin side of Fig. 7), that is, if the fiber density on the clothing side is lower than on the fiber density on the skin side (see low fiber density section LD on clothing side of Fig. 7), when the protrusion-recess nonwoven fabric and the absorbing element 4 become joined, the low density section LD on the clothing side of the top sections 22T of the second protrusions 22 are crushed and become joined by compaction, the fiber density thus becoming higher at the top sections 22T of the second protrusions 22 after joining, and therefore specifically, the fiber density at the top sections 22T of the second protrusions 22 becomes higher than the fiber density surrounding the top sections 22T (see even higher density section MHD in Fig. 7), allowing body fluids such as urine and menstrual blood to be taken up more easily from the areas surrounding the top sections 22T of the second protrusions 22 through to the joining sections of the top sections 22T. In Fig. 7, the relationship between the fiber densities of each section is LD < HD < MHD.

Moreover, the shape of each protrusion of the protrusion-recess nonwoven fabric depends on the shapes of the through-holes of the pattern plate and the suction force of the suction drum, the temperature and wind speed of the warm air blasted from the air jet, the basis weight of the fiber matrix F2, the material of the fibers composing the fiber matrix F2, etc., and therefore the shapes of the protrusions can be adjusted as desired by appropriate setting of these factors.

As shown in Fig. 5, the protrusion-recess fiber matrix F3 having a protrusion-recess form shaped by the suction drum 92 and air jet nozzle 94 is then conveyed to a heat setting machine 95 by a conveying apparatus such as a belt conveyor DB, and subjected to heat setting in the heat setting machine 95. The heat setting fixes the protrusion-recess form shaped in the protrusion-recess fiber matrix F3, and imparts flexibility to the protrusion-recess nonwoven fabric.

When heat setting of the protrusion-recess fiber matrix F3 has been completed, the protrusion-recess nonwoven fabric to be used as the front sheet 2 of the absorbent article of the present invention is complete. The completed protrusion-recess nonwoven fabric may be cut to a desired size, according to the form in which it is to be used.

The absorbing element to be used in the absorbent article of the present invention will now be described. The absorbing element to be used in the absorbent article of the present invention is not particularly restricted so long as it has a function of absorbing and retaining body fluids such as urine and menstrual blood, and any absorbing element employed in the prior art may be used, but from the viewpoint of comfort during wear, for example, it is preferably one with bulk, resistance to deformation and low chemical irritation. Examples of such absorbing elements include absorbent cores including fiber materials such as fluffy pulp, spunbond nonwoven fabrics, airlaid nonwoven fabrics, thermoplastic fibers and polymer absorbents such as super-absorbent polymers (SAP), at least partially covered by an absorbent core cover sheet comprising a liquid-permeable sheet such as a tissue sheet or liquid-permeable nonwoven fabric, hydrophilic nonwoven fabric or the like. The absorbent core does not have to include a super-absorbent polymer, and for example, any of the aforementioned fiber materials alone may be covered with a tissue and used as the absorbent core.

Also, instead of fluffy pulp or the like, a chemical pulp or artificial cellulose fiber such as cellulose fiber, rayon or acetate; a fiber network absorbent body employing synthetic fiber such as polyolefin, polyester or polyamide (including composite fiber); or a foamed absorbent body employing a foam material such as polyurethane, may be used as the fiber material of the absorbent core.

The basis weight of the fiber material is not particularly restricted, but from the viewpoint of the absorption property for body fluids such as urine and menstrual blood, it may be, for example, 50 g/m² to 1000 g/m², preferably 100 g/m² to 800 g/m² and more preferably 150 g/m² to 700 g/m².

The super-absorbent polymer to be used in the absorbent core is essentially water-insoluble, having a three-dimensional network structure with the water-soluble polymer in appropriate crosslinkage. Also, the super-absorbent polymer is preferably one that can absorb water up to at least 20 times, preferably 30 to 60 times and more preferably several hundred to a thousand times its own weight, and that does not exude water that has already been absorbed, even under some degree of stress. Examples of such super-absorbent polymers include starch-based polymers, crosslinked carboxymethylated cellulose, acrylic acid-based polymers including polymers or copolymers of acrylic acid or alkali metal salt acrylates, and amino acid-based polymers. The form of the super-absorbent polymer is not particularly restricted, and for example, it may be in the form of particulates, powder, masses, secondary particle aggregates, filaments or the like. It is preferably in the form of particulates, powder or filaments, and more preferably particulates with a particle size of 1 to 1000 µm and even more preferably particulates with a particle size of 10 to 500 µm.

The basis weight of the super-absorbent polymer is not particularly restricted, but from the viewpoint of the absorption property for body fluids such as urine and menstrual blood, it may be, for example, 50 g/m² to 1000 g/m², preferably 80 g/m² to 800 g/m² and more preferably 100 g/m² to 700 g/m². Also, from the viewpoint of the absorption property for body fluids such as urine and menstrual blood, the mass ratio of the super-absorbent polymer may be, for example, 10% to 100%, preferably 20% to 80% and more preferably 30 to 65%, with 100% as the fiber material.

The absorbent core cover sheet that at least partially covers the absorbent core may be constructed using a liquid-permeable sheet, such as an air-through nonwoven fabric, spunbond nonwoven fabric, point bond nonwoven fabric, hydrophilic-treated porous plastic film, liquid-permeable plastic film or the like.
Particularly when the liquid-permeable sheet is to be provided between a front sheet and absorbent core, the liquid-permeable sheet is preferably one with a higher fiber density than the front sheet. If such a liquid-permeable sheet is provided between the front sheet and the absorbent core, it will be able to improve the liquid drain property and reduce rewetting. Furthermore, if the fiber density of the liquid-permeable sheet is higher than the fiber density of the top sections of the second protrusion of the front sheet (that is, the fiber density of the top sections of the second protrusions is lower than the fiber density of the liquid-permeable sheet), body fluids such as urine and menstrual blood discharged onto the front sheet will easily migrate into the absorbent core.

The size of the fibers composing the liquid-permeable sheet is preferably 0.01 to 20 dtex and more preferably 1 to 10 dtex. If the size is too high the number of fibers will be reduced, making it difficult to hold the polymer absorbent between the fibers, while also tending inhibit swelling of the polymer absorbent (that is, reducing absorption). Conversely, if the size is too low, the rigidity of the fibers themselves will be reduced, making it difficult to maintain the basic structure of the liquid-permeable sheet made of the fibers. Furthermore, the fiber lengths of the fibers composing the liquid-permeable sheet are preferably 30 mm to 80 mm and more preferably 40 mm to 70 mm. If the fiber lengths are too long it will be difficult to manage the fibers, and conversely if the fiber lengths are too short, there will be fewer heat-fused sections, making it difficult to maintain the basic structure of the liquid-permeable sheet made of the fibers.

The basis weight of the liquid-permeable sheet is not particularly restricted, but from the viewpoint of the liquid drain property and strength, rewetting property, etc., it may be 5 g/m² to 50 g/m², for example, and is preferably 5 g/m² to 30 g/m² and more preferably 8 g/m² to 25 g/m².

The liquid-permeable sheet may cover the absorbent core as one sheet; however, preferably two or more sandwich the absorbent core to cover it. If the absorbent core does not deform, the absorbent core does not need to be covered by the liquid-permeable sheet.

Also, the absorbent core cover sheet may be composed of two or more liquid-permeable sheets at least in the region located between the front sheet and the absorbent core. In this case, the two or more liquid-permeable sheets are preferably disposed so that the fiber density gradually reduces from the liquid-permeable sheet on the skin side toward the liquid-permeable sheet on the clothing side. If two or more liquid-permeable sheets are disposed in this manner, it is possible to effectively prevent rewetting from the absorbent core, compared to an absorbent core cover sheet made of one liquid-permeable sheet.

The shape, construction and size of the absorbing element may be appropriately modified within a range that satisfies the absorption required as an absorbent article and comfort when worn.

A method of joining the front sheet and the absorbing element will now be described. The method of joining the front sheet and the absorbing element is not particularly restricted so long as it allows planar joints to be formed so that the forms of the joining sections are planar, and there may be employed any desired joining methods used in the prior art, including heat-fusing such as heat embossing, ultrasonic embossing or high-frequency embossing; bonding by an adhesive such as a hot-melt adhesive; or mechanical joining utilizing physical engagement, but from the viewpoint of fluid migration properties, bonding strength, feel, production equipment and easy management, heat-fusing by heat embossing or bonding by an adhesive such as a hot-melt adhesive is preferred. By using such joining means, it is possible to more reliably and more easily obtain structures for the joining sections that have excellent fluid migration properties. In addition, by employing joining means used in the prior art, such as embossing or an adhesive, it is possible to avoid complicating the production equipment or requiring construction of new production equipment.

Furthermore, when the joining is by heat embossing, a heat embossing apparatus comprising an embossing roll having a protrusion-recess form with a prescribed pattern on the peripheral surface and an anvil roll having a smooth peripheral surface, may be used and the front sheet and absorbing element introduced in an overlaid state between the two rolls, and pressing performed with the two rolls, to partially join the front sheet and the absorbing element.

Each roll may be adjusted to their respective prescribed temperatures, and the interval between the rolls may also be appropriately adjusted. The heating temperature for the embossing rolls is not particularly restricted, but preferably it is a temperature that at least partially melts the material facing the bonding surface of at least one of the front sheet and the absorbing element. From the viewpoint of fluid migration property, bonding strength and feel at the joining sections after joining, the heating temperature is in the range of 50°C to 300°C, for example, and is preferably 60°C to 200°C, more preferably 70°C to 150°C and even more preferably 80°C to 130°C.

The linear pressure between both rolls is not particularly restricted, but it is preferably set to be a pressure so that the low fiber density sections LD at the top sections of the second protrusions of the protrusion-recess nonwoven fabric composing the front sheet can be crushed to form planar joints, as described above. From the viewpoint of fluid migration properties, bonding strength and feel at the joining sections, the linear pressure may be in the range of, for example, 1 N/cm to 1000 N/cm, and is preferably 10 N/cm to 800 N/cm, more preferably 30 N/cm to 600 N/cm and even more preferably 50 N/cm to 400 N/cm.

There are no particular restrictions on the shapes of the joining sections in a plane view (or the shapes of the protrusions of the corresponding embossing roll), and for example, they may be circular, elliptical, rectangular, square, rhomboid or polygonal shapes, in a plane view. Also, the pattern in which the joining sections are arranged is not particularly restricted, but from the viewpoint of bulk, feel and strength after joining, and fluid migration properties of the joining sections, it is preferably a zigzag arrangement, at a pitch of, for example, 1 mm to 30 mm, preferably 3 mm to 10 mm and more preferably 4 mm to 7 mm.

For bonding with an adhesive, after the adhesive such as a hot-melt adhesive has been supplied in a prescribed pattern onto the clothing side of the front sheet and/or the skin side of the absorbing element using desired means such as coating, the front sheet and absorbing element may be overlaid either under pressure or without pressure, to partially bond them together.

There are no particular restrictions on the adhesive and any desired adhesive used in the prior art may be employed, but it is preferably a hot-melt adhesive from the viewpoint of fluid migration properties, bonding strength, feel, productivity and availability. Such a hot-melt adhesive is not particularly restricted, and examples include olefin-based adhesives such as polyethylene, polypropylene and ethylene-α-olefin copolymers; ethylene-vinyl acetate copolymer-based adhesives; polyamide-based adhesives; thermoplastic elastomer-based adhesives such as styrene-butylene-styrene copolymer and styrene-isoprene-styrene copolymer; and reactive hot-melt advantages such as moisture curing urethane prepolymers.

The prescribed pattern in which the adhesive is applied is not particularly restricted, and for example, it may be formed by σ-coating, spiral coating, coater application or the like, and it may be a dot pattern of various shapes including circular, elliptical, rectangular, square, rhomboid or polygonal shapes, in a plane view. The pattern may also be a lattice arrangement at a pitch of, for example, 1 mm to 30 mm, preferably 3 mm to 10 mm and more preferably 4 mm to 7 mm, from the viewpoint of bulk, feel and strength after joining, and the fluid migration properties of the joining sections.

Joining of the front sheet and absorbing element may be accomplished by any joining method without limitation to the methods mentioned above, and according to the present invention, it is indispensable for the joining sections at the top sections of the second protrusions of the front sheet to be joined in a planar form, i.e. to be "planar joints".

The term "planar joint" used through the present description means a form of joining in which at least portions of the top sections of the second protrusions on the front sheet, and especially the apex portions, infiltrate between the fibers composing the absorbing element, and especially the absorbent core cover sheet, with a joining area percentage of 4.0% or greater, as described below.

The term "joining area percentage", as used herein, refers to the area ratio of the joining sections per unit area of the overlapping region in which the front sheet and absorbing element overlap, in a plane view. The joining area percentage can be measured by a method using a scanning electron microscope, described below. If the joining area percentage is 4.0% or greater, preferably 5.0% or greater and more preferably 5.2% or greater, it will be possible to further improve the fluid migration property. Also, from the viewpoint of at least bulk and feel, the joining area percentage is preferably no greater than 60%, more preferably no greater than 40% and even more preferably no greater than 20%.

In the absorbent article of the present invention, as mentioned above, the front sheet used is preferably a protrusion-recess nonwoven fabric wherein the fiber density on the skin side is higher than the fiber density on the clothing side, at the top sections of the first or second protrusions. When such a protrusion-recess nonwoven fabric is used, as shown in Fig. 7 and Fig. 8, the fiber density on the clothing side is lower than the fiber density on the skin side at the top sections 22T of the second protrusions 22 of the front sheet 2, and therefore when the front sheet 2 and the absorbing element 4 are joined, the low fiber density sections LD are crushed and joined by compaction at the top sections 22T, and the fiber density of the top sections 22T of the second protrusions 22 after joining is higher than the fiber density of the areas surrounding the top sections 22T (see even higher fiber density section MHD portions in Fig. 7 and Fig. 8), thereby allowing body fluids such as urine and menstrual blood to be more easily taken up from the areas surrounding the top sections 22T of the second protrusions 22 to the joining sections of the top sections 22T.

Moreover, if the overlapping region where the front sheet and the absorbing element overlap in a plane view is divided into 3 equal portions in the widthwise direction of the absorbent article, according to a different embodiment of the present invention, preferably the front sheet and the absorbing element are joined in such a manner that the joining area percentage of the joining sections in the left and right regions in the widthwise direction is higher than the joining area percentage of the joining sections at the center region. If the joining area percentage in the left and right regions in the widthwise direction is higher than in the center region, then it will be possible to effectively prevent seepage of body fluids such as urine and menstrual blood from the edges in the widthwise direction of the absorbing element, while also reducing infiltration of body fluid at the center region compared to the left and right regions, thereby allowing absorption of the body fluid to be spread out over a wider region, and allowing the use efficiency of the absorbing element to be drastically improved.

According to yet another embodiment of the present invention, the front sheet is preferably joined with the absorbing element in such a manner that it does not cover the side edge sections on both sides extending in the lengthwise direction of the absorbent article in the absorbing element (that is, the front-back direction of the wearer). If the front sheet is joined in this manner, it will be possible to ensure excellent fluid migration properties by the front sheet, while avoiding discomfort for the wearer at the sections in sliding contact with the areas around the legs, such as the thighs of the wearer.

The present invention can also be suitably used in various absorbent articles such as incontinence pads, sanitary napkins and panty liners in addition to the embodiment of a disposable diaper described above. However, since the absorbent article of the present invention has a soft cushioning property, satisfactory restoration when pressed and excellent scavenging properties for excreta, as well as excellent fluid migration properties from the front sheet to the absorbing element, it is especially preferred for use in an absorbent article such as a disposable diaper, incontinence pad or sanitary napkin that absorbs large amounts of fluids.

The absorbent article of the present invention is not restricted to the embodiment described above or the examples which follow and appropriate modifications may be incorporated within a range that is not outside of the object and gist of the present invention.

### Examples

The present invention will now be explained in more detail based on examples, with the understanding that the present invention is not limited to the examples.

In order to examine the effect of the joined state between a protrusion-recess nonwoven fabric composing a front sheet and a nonwoven fabric composing a liquid-permeable sheet for an absorbing element (hereunder referred to as "absorbing element side nonwoven fabric") on the absorption behavior of the absorbent article, a layered body sample was fabricated having a protrusion-recess nonwoven fabric and a nonwoven fabric on the absorbing element side joined as described below.

### 1) Fabrication of protrusion-recess nonwoven fabric

For fabrication of a protrusion-recess nonwoven fabric, first composite fibers (fibers F1) having a size of 1.3 dtex and having a core-sheath structure of core/sheath = PET/HDPE were opened with an opener, and then forming of the fibers was carried out with a carding machine set for a prescribed basis weight. The formed carded web (fiber matrix F2) was conveyed by a mesh conveyor and attached onto the outer peripheral surface of a suction drum and supplied onto a pattern plate rotating together with the suction drum, and while the suction drum was rotated, the carded web was suctioned by negative pressure from the suction holes formed on the outer peripheral surface of the suction drum, the carded web being blasted from air jet nozzles situated at a prescribed location below the suction drum, with warm air at 140°C at a wind speed of 33.3 m/sec, causing the carded web to run along the pattern plate and forming a protrusion-recess form with a prescribed pattern in the carded web. Also, the web on which the protrusion-recess form had been formed (the protrusion-recess fiber matrix F3) was conveyed to the heat setting machine and subjected to heat setting under warm air conditions with a temperature of 133°C and a wind speed of 0.9 m/sec, and it was wound up to obtain a protrusion-recess nonwoven fabric. The basis weight of the obtained protrusion-recess nonwoven fabric was 30 g/m².

### 2) Fabrication of layered body sample with protrusion-recess nonwoven fabric and absorbing element side nonwoven fabric joined

As the absorbing element side nonwoven fabric there was used a nonwoven fabric with a basis weight of 20 g/m², formed using composite fibers having a size of 2.2 dtex, and a core-sheath structure of core/sheath = PET/HDPE. The previously fabricated protrusion-recess nonwoven fabric and this absorbing element side nonwoven fabric were joined by heat embossing or a hot-melt adhesive (HMA) under the conditions of each of the examples and comparative examples described below, to fabricate layered body samples for Examples 1 and 2 and Comparative Examples 1 to 6.

The joined state of each of the layered body samples obtained in this manner was confirmed by a scanning electron microscope, the joining region (a1), joining area percentage (a2), liquid permeation rate and drain speed were measured, and the absorption behavior of each layered body sample was compared. The conditions for photographing the joining sections with the scanning electron microscope and the measuring conditions for the joining region (a1) and joining area percentage (a2) were as follows.

### 3) Measurement of joining region (a1)

A cut surface at a joining section between the protrusion-recess nonwoven fabric and the absorbing element side nonwoven fabric was photographed at a magnification allowing observation of the total joining section by the scanning electron microscope (20-100x), and the joining region (a1) was determined by the scale of the taken photograph.

### 4) Measurement of joining area percentage (a2)

When the joining means was embossing, since the tips of the protrusions of the embossing have slightly rounded shapes, the joining area percentage (a2) was determined by calculating one joining area with the joining region (a1) measured as described above as the diameter, and calculating the joining area percentage (a2) by applying this to the prescribed emboss pattern. The joining area percentage (a2) does not have to depend on the emboss pattern, and the shapes of the protrusions may also be circular, elliptical, rectangular or the like. When the joining means was HMA, since the shapes of the tips of the protrusions of the protrusion-recess nonwoven fabric had slightly rounded shapes, one joining area was calculated with the joining region (a1) measured as described above as the diameter, and the joining area percentage (a2) was calculated by applying this to the prescribed protrusion-recess pattern.

### 5) Measurement of liquid permeation rate and drain speed

For measurement of the liquid permeation rate and drain speed, first the surface material was removed from a baby paper diaper, Moony "Air-Fit" S size, marketed by Unicharm Corp., and a layered body sample fabricated as described above was attached to the section from which the surface material had been removed, to prepare a sample for evaluation testing of the absorption property. The sample for evaluation testing of the absorption property was used for the following absorption property evaluation test. For the absorption property evaluation test, 40 ml of artificial urine was dropped once, and the time until all of the artificial urine migrated into the protrusion-recess nonwoven fabric was measured and the measured time was recorded as the liquid permeation rate. Similarly, the time until the artificial urine penetrated the protrusion-recess nonwoven fabric and completely migrated to the absorbing element side nonwoven fabric was calculated, and the calculated time was recorded as the drain speed. The artificial urine was prepared by dissolving 200 g of urea, 80 g of sodium chloride, 8 g of magnesium sulfate, 3 g of calcium chloride and approximately 1 g of dye (Blue #1) in 10 L of ion-exchanged water.

### 6) Measurement of fiber density

Measurement of the fiber density was conducted in the same manner as the method described in Japanese Unexamined Patent Publication No. 2012-144835 (see paragraph [0041]). Specifically, a cut surface of the nonwoven fabric was observed in an enlarged view using a scanning electron microscope (for example, a VE-7800 Real Surface View microscope by Keyence Corp.), with the center in the thickness direction of the nonwoven fabric as the center of observation (that is, observation at a magnification allowing about 20 to 70 fiber cross-sections to be observed, which is normally a magnification of about 20x to 100x), the number of cut cross-sections of fibers per fixed area (about 2 mm²) was counted, and the number of cut cross-sections was converted to the number of cut cross-sections of fibers per 1 mm² area, and used as the fiber density. This fiber density measurement was carried out at 3 locations, and the mean value was recorded as the fiber density for the sample.

### Example 1

A protrusion-recess nonwoven fabric fabricated as described above and an absorbing element side nonwoven fabric that had been spiral coated with an HMA were overlaid and bonded to integrate them. The joined state of the obtained layered body sample was confirmed by a scanning electron microscope, and the joining region (a1), joining area percentage (a2), liquid permeation rate and drain speed were measured. An electron micrograph of the layered body sample of Example 1 is shown in Fig. 9, and the joining region (a1) and other measurement results are shown in Table 1.

### Example 2

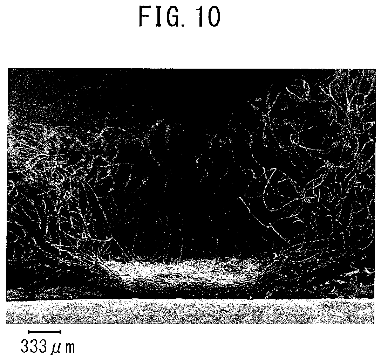

A layered body sample was fabricated in the same manner as Example 1, except that instead of a bonding method with HMA as the joining means there was employed an embossing method using an embossing roll heated to 110°C, comprising a plurality of pins arranged in a zigzag pattern with a pitch of 6.9 mm in the MD direction and a pitch of 4 mm in the CD direction. The joined state of the obtained layered body sample was confirmed by a scanning electron microscope, and the joining region (a1), joining area percentage (a2), liquid permeation rate and drain speed were measured. An electron micrograph of the layered body sample of Example 2 is shown in Fig. 10, and the joining region (a1) and other measurement results are shown in Table 1.

### Comparative Example 1

A layered body sample was fabricated for Comparative Example 1 in the same manner as Example 1, except that instead of a protrusion-recess nonwoven fabric there was used a flat nonwoven fabric without a protrusion-recess form. For fabrication of the flat nonwoven fabric, first synthetic fibers having a size of 1.3 dtex and having a core-sheath structure of core/sheath = PET/HDPE were opened with an opener, and forming of the fibers was carried out with a carding machine set for a prescribed basis weight. Also, the formed carded web was conveyed to a heat setting machine set to a prescribed temperature with a mesh conveyor, heat setting was carried out under warm air conditions with a temperature of 133°C and a wind speed of 0.9 m/sec, and it was then wound up to fabricate a flat nonwoven fabric. The basis weight of the obtained flat nonwoven fabric was 30 g/m².

The flat nonwoven fabric obtained in this manner and an absorbing element side nonwoven fabric were bonded by an HMA method in the same manner as Example 1, and integrated. Because Comparative Example 1 had two flat nonwoven fabrics bonded together, the joining area percentage of the layered body sample was 100%, and therefore only the liquid permeation rate and drain speed were measured for this layered body sample. Table 1 shows the measurement results including the liquid permeation rate for the layered body sample of Comparative Example 1.

### Comparative Examples 2 to 6

Layered body samples with different joining areas were fabricated for Comparative Examples 2 to 6 in the same manner as Example 1 or 2, except that the joining states were point joints (that is, joining areas of less than 4.0%). The joined state of each obtained layered body sample was confirmed by a scanning electron microscope, and the joining region (a1), joining area percentage (a2), liquid permeation rate and drain speed were measured. Table 1 shows the measurement results including the joining region (a1) for the layered body samples of Comparative Examples 2 to 6.

[Table 1]

**Table 1**

| | | Example 1 | Example 2 | Comparative Example 1 | Comparative Example 2 | Comparative Example 3 | Comparative Example 4 | Comparative Example 5 | Comparative Example 6 |
|---|---|---|---|---|---|---|---|---|---|
| Joint state | | Planar joint | Planar joint | | Point joint | Point joint | Point joint | Point joint | Point joint |
| Protrusion-recess nonwoven fabric forming conditions | Temperature (°C) | 140 | 140 | | 140 | 140 | 140 | 140 | 140 |
| | Wind speed (m/sec) | 33.3 | 33.3 | | 33.3 | 33.3 | 33.3 | 33.3 | 33.3 |
| Heat setting conditions | Temperature (°C) | 133 | 133 | 133 | 133 | 133 | 133 | 133 | 133 |
| | Wind speed (m/sec) | 0.9 | 0.9 | 0.9 | 0.9 | 0.9 | 0.9 | 0.9 | 0.9 |
| Protrusion-recess nonwoven fabric basis weight (g/m²) | | 30 | 30 | 30 | 30 | 30 | 30 | 30 | 30 |
| Protrusion-recess nonwoven fabric thickness (mm) | | 2.5 | 2.5 | 2.3 | 2.5 | 2.5 | 2.5 | 2.5 | 2.5 |
| Absorbing element side nonwoven fabric basis weight (g/m²) | | 20 | 20 | 20 | 20 | 20 | 20 | 20 | 20 |
| Absorbing element side nonwoven fabric thickness (mm) | | 0.4 | 0.4 | 0.4 | 0.4 | 0.4 | 0.4 | 0.4 | 0.4 |
| Joining method | | HMA | Embossing | HMA | Embossing | HMA | HMA | Embossing | Embossing |
| Joining region (a1) (µm) | | 3182.6 | 1922.5 | 100,000 | 99.5 | 460.0 | 1611.3 | 506.0 | 932.8 |
| Joining area (mm²) | | 7.96 | 2.90 | 10,000 | 0.01 | 0.17 | 2.04 | 0.20 | 0.68 |
| Joining area percentage (a2) (%) | | 14.36 | 5.24 | 100 | 0.01 | 0.30 | 3.68 | 0.36 | 1.23 |
| Liquid permeation rate (sec) | | 6.9 | 6.7 | 7.3 | 11.3 | 9.8 | 9.2 | 10.3 | 9.8 |
| Drain speed (sec) | | 28.6 | 23.9 | 38.6 | 36.7 | 44.5 | 36.0 | 29.9 | 27.3 |

As shown in Table 1, the layered body samples of Examples 1 and 2 which were joined by planar joints had notably increased liquid permeation rates and drain speeds compared to the layered body samples of Comparative Examples 2 to 6 which were joined by point joints. In addition, surprisingly, the layered body samples of Examples 1 and 2 had even higher liquid permeation rates and drain speeds than the layered body sample of Comparative Example 1, which had a joining area percentage of 100% (that is, entire surface joining). This supports the conclusion that when the fiber density at the top sections of the second protrusions of the protrusion-recess nonwoven fabric after joining is higher than the fiber density surrounding the top sections, fluids are more easily taken up from the areas surrounding the top sections to the joining sections of the top sections. Moreover, based on the measurement results for the liquid permeation rate and drain speed with the layered body samples of Examples 1 and 2, it is seen that a sample joined by embossing as with the layered body sample of Example 2 exhibits excellent absorption properties even with a relatively low joining area percentage, because the embossing roll pins compact the fibers so as force them from the front sheet to the absorbing element side nonwoven fabric.

### Reference Sign List

- 1: Disposable diaper
- 2: Front sheet
- 21: First protrusion
- 21T: Top section of first protrusion
- 22: Second protrusion
- 22T: Top section of second protrusion
- 3: Back sheet
- 4: Absorbing element
- 9: Production equipment
- 91: Carding machine
- 92: Suction drum
- 93: Pattern plate
- 94: Air jet nozzle
- 95: Heat setting machine
- F1: Fibers
- F2: Fiber matrix
- F3: Protrusion-recess fiber matrix

## Claims

1. An absorbent article having:
a front sheet with a protrusion-recess structure, to be used on a skin side of the absorbent article, the front sheet having a first protrusion that protrude to the skin side and a second protrusion that protrude to a clothing side on opposite side from the skin side, and
an absorbing element situated at a location overlapping with the front sheet in a plane view,
wherein the front sheet and the absorbing element are joined in a planar form at a joining section of a top section of the second protrusion.

2. The absorbent article according to claim 1, wherein the joining section with a planar form is formed by planar joint using embossing or an adhesive.

3. The absorbent article according to claim 2, wherein the adhesive is a hot-melt adhesive.

4. The absorbent article according to any one of claims 1 to 3, wherein the front sheet is a nonwoven fabric, and a fiber density of the top section of the second protrusion at the joining section is higher than a fiber density surrounding the top section.

5. The absorbent article according to any one of claims 1 to 4, wherein when an overlapping region where the front sheet and the absorbing element overlap in a plane view is divided into 3 equal sections in a widthwise direction of the absorbent article, a joining area percentage of the joining section in left and right regions in the widthwise direction is higher than the joining area percentage of the joining section in a center region.

6. The absorbent article according to any one of claims 1 to 5, wherein the first protrusion and the second protrusion have at least one shape selected from a group consisting of cylindrical, elliptic cylindrical, polygonal columnar, conical, pyramidal, truncated conical and truncated pyramidal shapes.

7. The absorbent article according to any one of claims 1 to 6, wherein the front sheet is a nonwoven fabric, the absorbing element includes an absorbent core and an absorbent core cover sheet comprising a liquid-permeable sheet that at least partially covers the absorbent core from the skin side and is joined to the top section of the second protrusion of the front sheet, and the fiber density of the liquid-permeable sheet is higher than the fiber density at the top section of the second protrusion.

8. The absorbent article according to any one of claims 1 to 7, wherein the absorbing element includes an absorbent core and an absorbent core cover sheet comprising a liquid-permeable sheet that at least partially covers the absorbent core and is joined to the top section of the second protrusion of the front sheet, the absorbent core cover sheet is composed of two or more liquid-permeable sheets at least in the region located between the front sheet and the absorbent core the two or more liquid-permeable sheets being disposed so that the fiber density is gradually lower from the liquid-permeable sheet on the skin side toward the liquid-permeable sheet on the clothing side.

9. The absorbent article according to any one of claims 1 to 8, wherein the front sheet is joined to the absorbing element so as not to cover side edge sections on both sides of the absorbing element extending in a lengthwise direction of the absorbent article.
